# EUROPEAN PATENT APPLICATION

(11) **EP 1 469 070 A1**
(43) Date of publication of application: **20.10.2004**
(21) Application number: 03008081.6
(22) Date of filing: 15.04.2003
(51) Int. Cl.: C12N 15/11

(54) **Livin-specific siRNAs for the treatment of therapy-resistant tumors**

(71) Applicant: Deutsches Krebsforschungszentrum Stiftung des öffentlichen Rechts, 69120 Heidelberg (DE)
(72) Inventor: Butz, Karin, Dr., 69493 Hirschberg (DE); Crnkovic-Mertens, Irena, Dr., 69124 Heidelberg (DE); Hoppe-Seyler, Felix, Prof.-Dr., 69118 Heidelberg (DE)
(74) Representative: Huhn, Michael, Dr.

(57) **Abstract**

Provided is the use of siRNAs which are specific for the inhibitor of apoptosis protein (IAP) livin to sensitize tumor cells for apoptosis by down-regulating livin expression.

SEQUENCE LISTING

<110> Deutsches Krebsforschungszentrum

<120> Livin-specific siRNAs for the treatment of therapy-resistant tumors

<130> DK62169EP

<160> 5

<170> PatentIn version 3.1

<210> 1
<211> 19
<212> RNA
<213> Homo sapiens

<400> 1
cugguucccc agcugucag 19

<210> 2
<211> 19
<212> RNA
<213> Homo sapiens

<400> 2
ggaagagacu uuguccaca 19

<210> 3
<211> 47
<212> DNA
<213> Homo sapiens

<220>
<221> gene
<222> (1)..(19)

<223>

<220>
<221> misc_feature
<222> (20)..(28)
<223> linker

<220>
<221> gene
<222> (29)..(47)
<223>

<400> 3
gtggttcccc agctgtcagt tcaagagact gacagctggg gaaccac 47

<210> 4
<211> 47
<212> DNA
<213> Homo sapiens

<220>
<221> gene
<222> (1)..(19)
<223>

<220>
<221> misc_feature
<222> (20)..(28)
<223> linker

<220>
<221> gene
<222> (29)..(47)
<223>

<400>  4
ggaagagact ttgtccacat tcaagagatg tggacaaagt ctcttcc                    47

<210>  5

<211>  47

<212>  DNA

<213>  Photinus pyralis


<220>

<221>  gene

<222>  (1)..(19)

<223>


<220>

<221>  misc_feature

<222>  (20)..(28)

<223>  linker


<220>

<221>  gene

<222>  (29)..(47)

<223>


<400>  5
catcacgtac gcggaatact tcaagagagt attccgcgta cgtgatg                    47

## Description

The present invention relates to the use of siRNAs which are specific for the inhibitor of apoptosis protein (IAP) livin (ML-IAP, KIAP) to sensitize tumor cells for apoptosis by down-regulating livin expression. Thus, a novel tool for the treatment of therapy-resistant tumors is provided.

Tumor cells are typically characterized by their failure to undergo so-called programmed cell death or apoptosis, which allows their survival and continuous proliferation under the influence of abnormal growth stimuli. Moreover, apoptosis deficiency is considered to be a major cause for the therapeutic resistance of tumors in the clinic, since many chemo- and radiotherapeutic agents act through induction of apoptosis. An increasing understanding of the regulatory circuits contributing to the apoptosis resistance of cancer cells may therefore provide a rational basis for the development of novel therapeutic strategies, e.g. by specifically interfering with the activity of anti-apoptotic factors in tumor cells. Thus, the problem underlying the present invention refers to the identification of compounds or molecules that specifically modulate distinct steps in the apoptosis pathway by interfering with the activity of anti-apoptotic factors.

Apoptosis pathways involve diverse groups of molecules. One set of mediators implicated in apoptosis are so-called caspases, cysteine proteases that cleave their substrate specifically at aspartate residues. Caspases convey the apoptotic signal in a proteolytic cascade, with caspases cleaving and activating other caspases which subsequently degrade other cellular targets eventually resulting in cellular breakdown. In human tumors, a high expression of anti-apoptotic factors is commonly found and contributes to those neoplastic cell expansion and resistance to the therapeutic action of chemotherapeutic drugs. One group of structurally related proteins with anti-apoptotic properties is the inhibitor of apoptosis protein (IAP) family. IAPs bind to early active caspases, thereby preventing the ongoing of the apoptosis process. They are expressed at high levels in many tumors and, by inhibition of caspases, contribute to the resistance of cancers against apoptosis induction. Examples of IAPs include NAIP, XIAP (hILP), cIAP-1, cIAP-2, BIRC5 (survivin), TIAP, and Apollon. One rather novel member of this family is the livin/ML-IAP/KIAP protein, which is hereinafter referred to as livin. The ectopic expression of livin can block apoptosis induction by a variety of pro-apoptotic stimuli. This appears to be mediated, at least in part, by inhibiting capase-3 and -9. The livin gene exhibits a restricted expression pattern, since it has been found to be expressed in certain tumor cells, including melanoma or HeLa cervical carcinoma cells, but not, or to substantially lesser amounts, in most normal adult tissues. Its expression in tumor cells suggests that livin, as it is assumed for other IAPs, may contribute to tumorigenesis by causing apoptosis resistance. Consequently, inhibition of livin expression may represent an interesting therapeutic strategy to specifically correct the apoptosis deficiency of tumor cells.

Generally, the inhibition of protein expression can be exerted by several different strategies. The patent application WO-A 00/77201 describes a variety of molecules, either on the nucleic acid or on the protein level, which are able to interfere with or down-regulate the expression of livin. For example, therapeutic antibodies against the livin protein can be used to antagonize endogenous livin and, thus, for the treatment of diseases associated with altered apoptosis, such as cancers. Furthermore, WO-A 00/77201 provides nucleic acid sequences that hybridize to the livin encoding DNA and mRNA, respectively. According to the cited patent application the nucleic acid molecules, particularly anti-sense molecules, can be used as tools for modulating the expression of the livin gene. For example, the expression of an anti-sense molecule representing the complete livin cDNA in anti-sense orientation led to a marked induction of apoptosis. This approach, though, bears the disadvantage that a regulated induction of apoptosis is not possible in cases of cancer where only a moderate apoptotic response is desired.

A promising tool to down-regulate and interfere with the expression of proteins has been developed according to the observation that double-stranded RNA molecules (dsRNA) with homology to a defined sequence within a gene acts as a "trigger" for post-transcriptional gene silencing (PTGS). The unique aspect of this process named "RNA interference" (RNAi) is its exquisite sequence-specificity, leading to the degradation of the targeted mRNA. RNAi has been originally discovered as a mechanism of PTGS in plants and animals (Fire et al., 1998, Nature, vol. 391, pages 806-811). In mammalian cells, RNAi can be achieved by transfecting 19-21 nucleotide (nt) small interfering siRNAs which are complementary to the mRNA sequence of a given target gene (Elbashir et al., 2001, Nature, vol. 411, pages 494-498), indicating that RNAi may serve as a powerful technology to specifically block the expression of target genes. As such, the use of siRNAs to inhibit the expression of defined target genes has obvious therapeutic potential.

The applicant has found that siRNAs being homologous to a distinct region on the livin cDNA resulted in an inhibition of livin gene expression which was associated with a strongly increased apoptotic response. The observed effect was enhanced in the presence of pro-apoptotic agents, indicating that the interference with livin leads to a sensitization for pro-apoptotic stimuli. In addition, the inventors circumvented the transient nature of transfected siRNAs and the requirements for their chemical synthesis by exploiting vector systems which are allow the expression of siRNAs.

Thus, a first aspect of the present invention refers to the use of a nucleic acid, preferably a RNA, containing the sequence of SEQ ID NO: 1, a fragment or derivative thereof, to prepare siRNA which sensitizes therapy-resistant tumor cells for apoptosis.

A second aspect of the present invention refers to the use of a nucleic acid, preferably a RNA containing the sequence of SEQ ID NO: 2, a fragment or derivative thereof, to prepare siRNA which sensitizes therapy-resistant tumor cells for apoptosis.

The sequence of SEQ ID NO: 1, which is preferably a RNA corresponds to nucleotides 611-629 of the coding sequence of the livin gene as disclosed by gene bank accession no. AF311388. The sequence of SEQ ID NO: 2 corresponds to nucleotides 648-666 according to the same gene bank accession number.

In the context of the present invention, the terms "fragment and derivative" refer to nucleic acids that may differ from the original nucleic acid in that they are extended or shortened on either the 5' or the 3' end, on both ends or internally, or extended on one end, and shortened on the other end, provided that the function of the resulting siRNA, namely the down-regulation of the target protein, is not abolished or inhibited. The terms "fragment and derivative" also refer to nucleic acids that may differ from the original nucleic acid in that one or more nucleotides of the original sequence are substituted by other nucleotides and/or (chemically) modified by methods available to the skilled artisan, provided that the function of the resulting siRNA is not abolished or inhibited.

The person of skill in the art knows how to prepare siRNA when the above disclosed nucleic acids, particularly RNAs, are provided. Briefly, the strands complementary to the nucleic acids of the present invention are synthesized by any available method and the complementary strands are annealed to the nucleic acid of the present invention under appropriate conditions. The annealing conditions, e.g. temperatures and incubation periods, may be adjusted according to the respective nucleic acid sequence.

In order to exert the desired function, i.e. the induction of apoptosis in therapy-resistant cells, the siRNAs prepared from the above nucleic acids of the present invention, are delivered into target cells.

There are several well-known methods of introducing nucleic acids into animal cells, any of which may be used in the present invention and which depend on the host. Typical hosts include mammalian species, such as humans, non-human primates, dogs, cats, cattle, horses, sheep, and the like. At the simplest, the nucleic acid can be directly injected into the target cell / target tissue. Other methods include fusion of the recipient cell with bacterial protoplasts containing the nucleic acid, the use of compositions like calcium chloride, rubidium chloride, lithium chloride, calcium phosphate, DEAE dextran, cationic lipids or liposomes or methods like receptor-mediated endocytosis, biolistic particle bombardment ("gene gun" method), infection with viral vectors, electroporation, and the like.

In a preferred embodiment of the present invention, the siRNAs are delivered to the cell by using liposomes. Another option may include the direct application of siRNAs by hydrodynamic, high pressure injection into the blood stream.

The effect of siRNAs, i.e. the reduction of the expression of a certain gene, is considered to be only transient when they are directly applied to cells as described supra. In order to achieve a stable production of siRNAs in therapy-resistant cells it can be advantageous if the nucleic acid, preferably a DNA, encoding the respective target siRNA is integrated in an expression vector. Providing suitable elements, as described hereinafter, the DNA is transcribed into the corresponding RNA which is capable of forming the desired siRNA.

The expression vector is preferably a eukaryotic expression vector, or a retroviral vector, a plasmid, bacteriophage, or any other vector typically used in the biotechnology field. If necessary or desired, the nucleic acid can be operatively linked to regulatory elements which direct the synthesis of a mRNA in pro- or eukaryotic cells. Such regulatory elements are promoters, enhancers or transcription termination signals, but can also comprise introns or similar elements, for example those, which promote or contribute to the stability and the amplification of the vector, the selection for successful delivery and/or the integration into the host's genome, like regions that promote homologous recombination at a desired site in the genome. For therapeutic purposes, the use of retroviral vectors has been proven to be most appropriate to deliver a desired nucleic acid into a target cell.

In a preferred embodiment of the present invention, a nucleic acid, preferably a DNA, which is suitable for the preparation of siRNA that induces apoptosis is introduced into a vector which allows for the production of a double-stranded (ds) RNA molecule. Such vectors are known to the person skilled in the art. To drive the expression of siRNAs these vectors usually contain RNA polymerase III promoters, such as the H1 or U6 promoter, since RNA polymerase III expresses relatively large amounts of small RNAs in mammalian cells and terminates transcription upon incorporating a string of 3-6 uridines. Type III promoters lie completely upstream of the sequence being transcribed which eliminates any need to include promoter sequence in the siRNA. If the DNA encoding the desired siRNA should be transcribed from one promoter, the preferred DNA should thus contain the desired coding region of the livin gene to be targeted as well as its complementary strand, wherein the coding and its complementary strand are separated by a nucleotide linker, allowing for the resulting transcript to fold back on itself to form a so-called stem-loop structure.

An example for such an expression vector which allows for the production of dsRNA directly in the target cell is the so-called pSUPER (Supression of Endogenous RNA). The vector itself and the mechanism how the dsRNA is produced by using said vector is described in Brummelkamp et al., 2002, Science, Vol. 296, pages 550-553. Another example of such a vector named p*Silencer* (Ambion) was developed by Sui et al., 2002, Proc. Natl. Acad. Sci. Vol. 99, pages 5515-5520.

Therefore, another object of the present invention is the use of a nucleic acid, preferably a DNA, containing the sequence of SEQ ID NO:3, a fragment or derivative thereof, to prepare siRNA which induces apoptosis in therapy-resistant cells. SEQ ID NO:3 comprises the DNA corresponding to the RNA depicted in SEQ ID NO:1, a linker, and the complementary strand to said DNA.

Furthermore, an object of the present invention is the use of a nucleic acid, preferably a DNA, containing the sequence of SEQ ID NO:4, a fragment or derivative thereof, to prepare siRNA which induces apoptosis in therapy-resistant cells. SEQ ID NO:4 comprises the DNA corresponding to the RNA depicted in SEQ ID NO:2, a linker, and the complementary strand to said DNA.

The linker is preferably 5 to 15 nucleotides in length, more preferably the linker is 7 to 12 nucleotides long and most preferably it is 9 nucleotides long. The linker can consist of any suitable nucleotide sequence. For the present invention, a linker comprising the nucleotides 20 to 28 of SEQ ID NOs: 3 and 4 represents a suitable linker.

It also contemplated in the present invention that the expression of the two complementary strands giving rise to a dsRNA is driven from two promoters, either the same or different. In this case, the nucleotide linker separating the two complementary strands would be omissible. It is further obvious to the one skilled in the art that in this case the DNAs coding for the two complementary siRNA strands can be present on one vector or on two vectors. An example for a vector by which siRNA is expressed from two promoters is described in Lee et al., 2002, Nature Biotechnology, Vol. 19, pages 500-505. For these purposes, nucleic acids containing the nucleotides 1-19 together with 29-47 of either SEQ ID NO:3 or 4 are inserted into the allocated sites of the vector(s). In the context of the present invention, the nucleic acids containing the nucleotides 1-19 or 29-47 of either SEQ ID NO:3 or 4 are regarded as fragments or derivatives, according to the above definition, of either SEQ ID NO:3 or 4.

The vector containing the DNAs of the present invention can be introduced into the target cell by any of the method described above. However, due to several limitations accompanied with the efficient delivery and/or toxic effects, the delivery of the nucleic acids of the present invention can be facilitated by the use of so-called translocating peptides. The use of such peptides are particularly preferred for therapeutic purposes. The peptides are usually linked to the nucleic acid to be delivered in a non-covalent manner.

In general, peptides are contemplated which mimic and act as efficiently as viruses for gene delivery without their limitations of inducing immune responses or being cytotoxic. Examples of peptides forming peptide-DNA complexes for an efficient delivery into a cell comprise DNA-condensing motifs such as polyamines and modifications thereof, active-targeting motifs such as RGD, endosomolytic peptides such as INF, JTS1 or GALA, and nuclear localization sequences (NLS), e.g. derived from the large tumor antigen of Simian 40 virus. An extensive list of translocating peptides and their proposes delivery mechanisms, all of which are contemplated within the scope of the present invention and incorporated herein by reference are described in Morris et al., 2000, Curr. Opin. Biotech., Vol. 8, pages 21 to 27.

A further object of the present invention refers to a method for down-regulating livin in therapy-resistant tumor cells in order to increase caspase-3-like activities and, thus, sensitize therapy-resistant tumor-cells for apoptosis. The method comprises contacting a therapy-resistant tumor cell with a siRNA containing the sequence of SEQ ID NOs: 1, 2, 3, and/or 4.

As indicated supra the siRNAs of the present invention sensitize tumor cells for apoptosis and, as a consequence, render such cells more susceptible to various treatment methods employed in tumor therapy.

Accordingly, the siRNAs as disclosed in the present invention can be used as a pharmaceutical, optionally in combination with radiation therapy and/or at least one active compound, for the treatment of therapy-resistant cancers. This is a further embodiment of the present invention.

First, the phrase "radiation therapy" refers to the use of electromagnetic or particulate radiation in the treatment of neoplasia. Radiation therapy is based on the principle that high-dose radiation delivered to a target area will result in the death of reproductive cells in both tumor and normal tissues. The radiation dosage regimen is generally defined in terms of radiation absorbed dose (rad), time and fractionation, and must be carefully defined by the oncologist. The amount of radiation a patient receives will depend on various consideration but the two most important considerations are the location of the tumor in relation to other critical structures or organs of the body, and the extent to which the tumor has spread. Examples of radiotherapeutic agents are provided in, but not limited to, radiation therapy and is known in the art (Hellman, Principles of Radiation Therapy, Cancer, in Principles and Practice of Oncology, 24875 (Devita et al., 4^{th} ed., vl, 1993). Recent advances in radiation therapy include three-dimensional conformal external beam radiation, intensity modulated radiation therapy (IMRT), stereotactic radiosurgery and brachytherapy (interstitial radiation therapy), the latter placing the source of radiation directly into the tumor as implanted "seeds". These newer treatment modalities deliver greater doses of radiation to the tumor, which accounts for their increased effectiveness when compared to standard external beam radiation therapy.

Ionizing radiation with beta-emitting radionuclides is considered the most useful for radiotherapeutic applications because of the moderate linear energy transfer (LET) of the ionizing particle (electron) and its intermediate range (typically several millimeters in tissue). Gamma rays deliver dosage at lower levels over much greater distances. Alpha particles represent the other extreme; they deliver very high LET dosage, but have an extremely limited range and must, therefore, be in intimate contact with the cells of the tissue to be treated. In addition, alpha emitters are generally heavy metals, which limits the possible chemistry and presents undue hazards from leakage of radionuclide from the area to be treated. Depending on the tumor to be treated all kinds of emitters are conceivable within the scope of the present invention.

Furthermore, the present invention encompasses types of non-ionizing radiation like e.g. ultraviolet (UV) radiation, high energy visible light, microwave radiation (hyperthermia therapy), infrared (IR) radiation and lasers. In a particular embodiment of the present invention UV radiation is applied.

Generally, radiation therapy can be combined temporally with other active compounds listed below to improve the outcome of treatment. There are various terms to describe the temporal relationship of administering radiation therapy together with other active compounds, and the following examples are the preferred treatment regimens and are generally known by those skilled in the art and are provided for illustration only and are not intended to limit the use of other combinations. Administration of radiation therapy with other active compounds can be "sequential", i.e. separately in time in order to allow the separate administration, "concomitant" which refers to the administration on the same day, and, finally, "alternating" which refers to the administration of radiation therapy on the days in which other active compounds would not have been administered.

The term "active compound" refers to a compound other than the nucleic acid which is able to induce apoptosis or which inhibits cell proliferation. Active compounds which are able to induce apoptosis are known to the person skilled in the art.

One class of active compounds are chemical compounds having a cytostatic or antineoplastic effect ("cytostatic compound"). Cytostatic compounds included in the present invention comprise, but are not restricted to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18-OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists.

In a preferred embodiment of the present invention the cytostatic compound is selected from the group consisting of cisplatin, doxorubicin and paclitaxel. Most preferred, the cytostatic compound is doxorubicin.

Another class of active compounds which can be used in the present invention are those which are able to sensitize for or induce apoptosis by binding to death receptors ("death receptor agonists"). Such agonists of death receptors include death receptor ligands such as tumor necrosis factor α (TNF-α), tumor necrosis factor β (TNF-β, lymphotoxin-α), LT-β (lymphotoxin-β), TRAIL (Apo2L, DR4 ligand), CD95 (Fas, APO-1) ligand, TRAMP (DR3, Apo-3) ligand, DR6 ligand as well as fragments and derivatives of any of said ligands. Preferably, the death receptor ligand is TNF-α.

Furthermore, death receptors agonists comprise agonistic antibodies to death receptors such as anti-CD95 antibody, anti-TRAIL-R1 (DR4) antibody, anti-TRAIL-R2 (DR5) antibody, anti-TRAIL-R3 antibody, anti-TRAIL-R4 antibody, anti-TRAIL-R5 (osteoprotegerin) antibody, anti-DR6 antibody, anti TNF-R1 antibody and anti-TRAMP (DR3) antibody as well as fragments and derivatives of any of said antibodies.

Finally, a class of active compounds which can be used in combination with the siRNAs of the present invention are peptides, proteins or small molecule inhibitors which negatively regulate or inhibit anti-apoptotic proteins. Examples of negatively regulating peptides include Smac/DIABLO, NRAGE and TAK1, fragments and derivatives thereof, which particularly inhibit IAPs. These peptides may be modified in a way that they can be rapidly internalized into tumor cells by cellular uptake. The modification can occur by attaching a carrier peptide that mediates said cellular uptake.

The nucleic acid of the present invention can be administered alone or in combination with radiation and/or one or more active compounds. The latter can be administered before, after or simultaneously with the administration of the nucleic acid. The dose of either the nucleic acid or the active compound as well as the duration and the temperature of incubation can be variable and depends on the target that is to be treated.

A further object of the present invention are pharmaceutical preparations which comprise an effective dose of at least one nucleic acid and/or at least one active compound and a pharmaceutically acceptable carrier, i.e. one or more pharmaceutically acceptable carrier substances and/or additives.

The pharmaceutical according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the pharmaceutical compositions can be carried out in a manner known per se. To this end, the nucleic acid and/or the active compound, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiological sodium chloride solution, alcohols such as ethanol, glycerol, polyols, sucrose, invert sugar, glucose, mannitol, vegetable oils, etc. It is also possible to lyophilize the nucleic acid and/or the active compound and to use the resulting lyophilisates, for example, for preparing preparations for injection or infusion. Suitable carriers for microcapsules, implants or rods are, for example, copolymers of glycolic acid and lactic acid.

The pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the nucleic acid, in combination with one or more active compounds to be administered, depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to the nucleic acid.

The nucleic acid according to the present invention, respectively the medicaments containing the latter, optionally in combination with one or more active compounds can be used for the treatment of all cancer types which are resistant to apoptosis due to the expression of livin.

Examples of such cancer types comprise neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, follicular thyroid carcinoma, anaplastic thyroid carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

Examples of cancer types where the use of the cytokine antagonists according to the present invention, respectively the medicaments containing the latter, is particularly advantageous include cervical carcinoma and melanoma.

### DESCRIPTION OF THE DRAWING

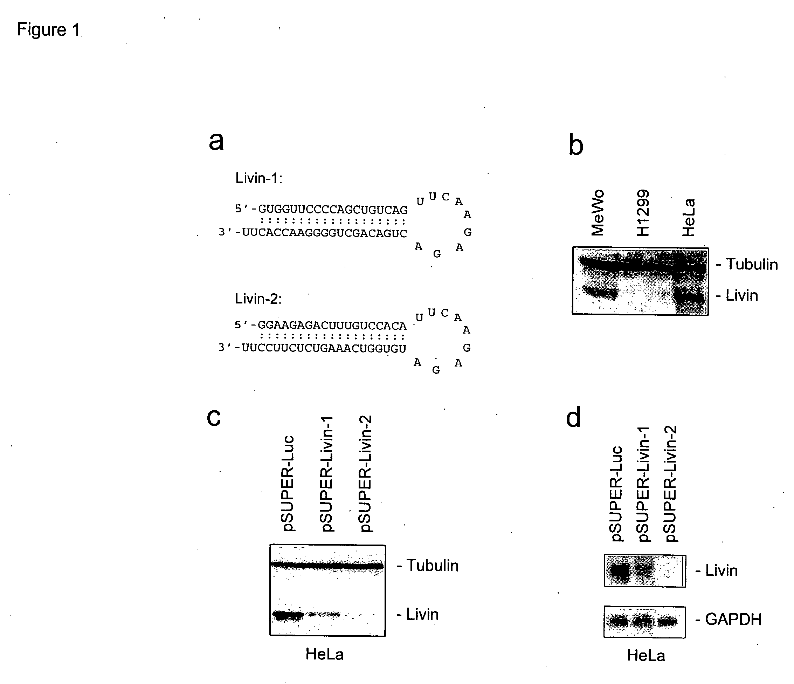

**Figure 1.** siRNA-mediated inhibition of Livin expression. **(a)** Predicted secondary structure of pSUPER-Livin-1 and pSUPER-Livin-2 transcripts. **(b)** Western blot analysis of Livin protein expression in MeWo melanoma, H1299 lung cancer and HeLa cervical carcinoma cells. Tubulin: detection of α-Tubulin protein expression to monitor equal loading between individual lanes. **(c)** Inhibition of Livin protein expression in HeLa cells by pSUPER-Livin-1 and pSUPER-Livin-2. Control vector pSUPER-Luc expresses siRNA targeting the *P. pyralis* luciferase gene. **(d)** Reduction of livin transcripts in HeLa cells by siRNA targeting of livin. Northern blot analysis of poly-A⁺-RNA isolated from HeLa cells transfected with pSUPER-Livin-1, pSUPER-Livin-2, or control transfected HeLa cells, respectively. GAPDH: detection of glycerylaldehyde-3-phosphate dehydrogenase transcripts.

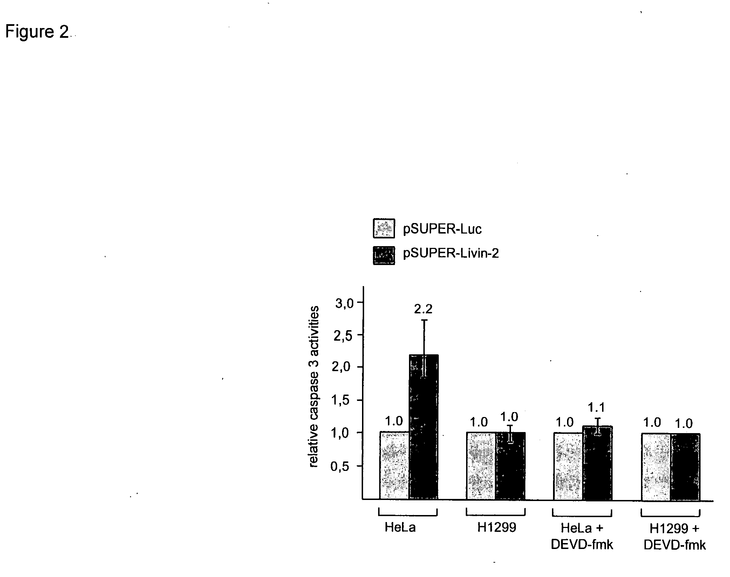

**Figure 2.** siRNA against Livin increases Caspase-3 activities in HeLa cells. Livin-positive HeLa and Livin-negative H1299 cells were transfected with either pSUPER-Livin-2 or control vector pSUPER-Luc. DEVD-pNA hydrolysis was measured in cytosolic extracts 48 hours post-transfection. Indicated are the Caspase-3 activities of pSUPER-Livin-2-transfected cells relative to control transfectants (pSUPER-Luc), arbitrarily set at 1.0. Values represent the means obtained from at least three independent transfections, error bars indicate the standard deviations. Inclusion of the specific Caspase-3 inhibitor DEVD-fink blocks pSUPER-Livin-2-induced hydrolysis of DEVD-pNA.

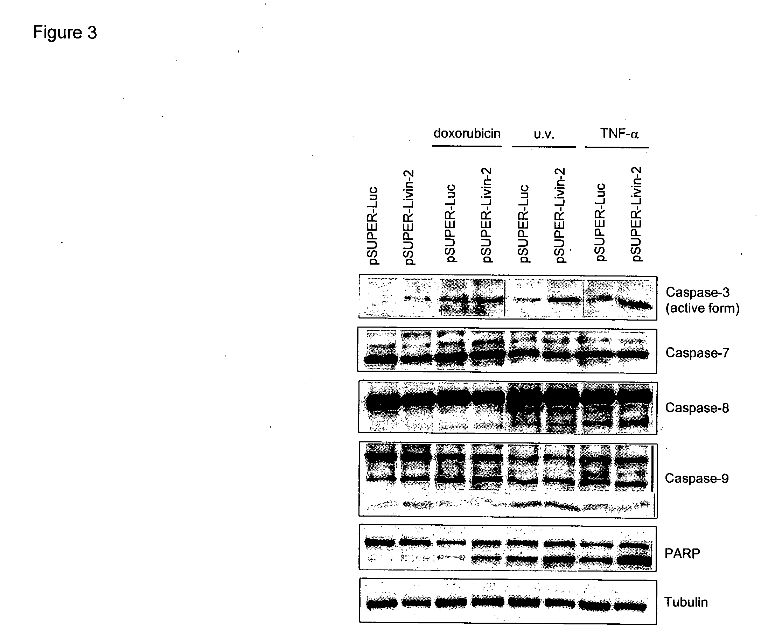

**Figure 3.** pSUPER-Luc-2 leads to promoted cleavage of Caspase-3 and Poly(ADP-ribose) polymerase (PARP). Western blots detecting Caspase and PARP expression patterns in pSUPER-Livin-2 or pSUPER-Luc transfected HeLa cells, either untreated, or following exposure to the pro-apoptotic stimuli doxorubicin, u.v.-irradiation, or TNF-α. Tubulin: detection of α-Tubulin protein expression to monitor equal loading between individual lanes.

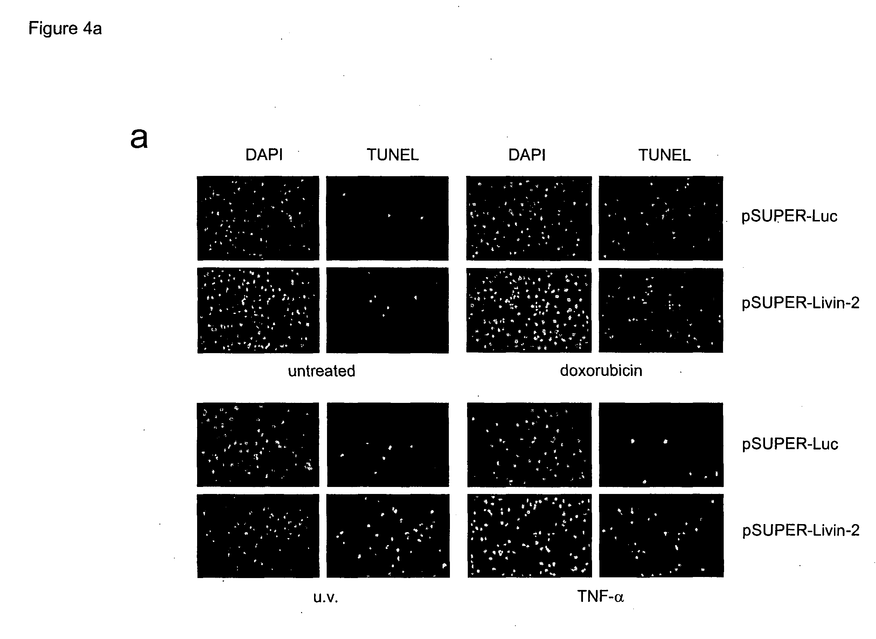

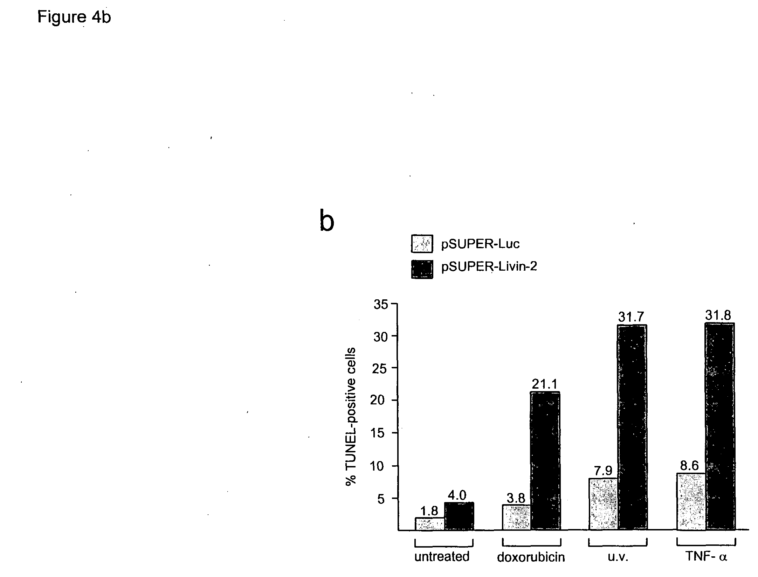

**Figure 4.** siRNA-mediated targeting of livin strongly sensitizes HeLa cells toward pro-apoptotic stimuli. HeLa cells were transfected with control vector pSUPER-Luc or pSUPER-Livin-2. Cells were either left untreated or exposed to doxorubicin, u.v.-irradiation, or TNF-α. (**a**) Total DNA was stained with DAPI, cells undergoing apoptosis were visualized by TUNEL. (**b**) Percentage of TUNEL positive cells.

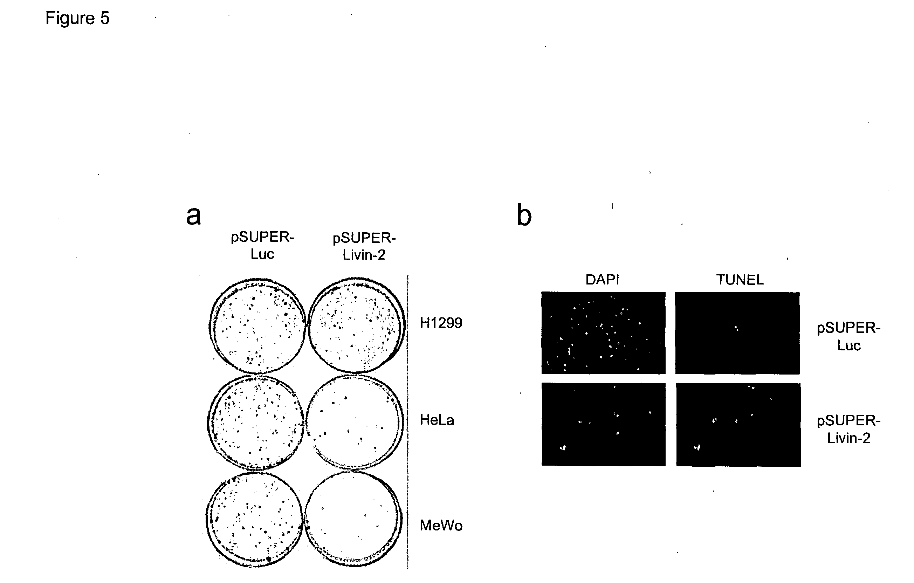

**Figure 5.** siRNA-mediated targeting of livin selectively blocks the growth of Livin-positive cancer cells through induction of apoptosis. (**a**) Colony formation assays of H 1299 cells (Livin-negative) and HeLa and MeWo cells (both Livin-positive). Cells were transfected with either pSUPER-Livin-2 or control vector pSUPER-Luc, together with pSV2Neo, and grown for 6-10 days in the presence of Geneticin to select for stable transfectants. Colonies were fixed with formaldehyde and stained with crystal violet. (**b**) After 4 days of selection, HeLa cells were investigated for signs of apoptosis. Please note that the reduction of cell numbers in the presence of pSUPER-Livin-2 is even more pronounced, since a five-fold lower number of pSUPER-Luc transfected control cells was plated for this figure, in order to avoid confluency. Total cellular DNA was stained with DAPI, cells undergoing apoptosis were visualized by TUNEL assay.

The invention is further illustrated by the following examples:

### EXAMPLES

### Example 1: Livin-1 and Livin-2 siRNAs reduce livin transcripts and inhibit endogenous livin protein expression

Two RNAi target regions, livin-1 and livin-2, were selected from the livin cDNA following the recommendations for the design of efficient siRNA sequences (Brummelkamp et al., 2002). Both sequences are present in the two splice forms of Livin, Livin α (KIAP) and Livin β (ML-IAP) known to the skilled artisan. They were introduced into the pSUPER vector system in the form of synthetic oligonucleotides, consisting of the respective 19 nt target sequences, separated by a 9 nt spacer from the reverse complement of the 19 nt target sequences, yielding pSUPER-Livin-1 and pSUPER-Livin-2. The predicted secondary structures of the plasmid-derived transcripts are depicted in Figure 1a.

HeLa cells were chosen for RNA interference, since they express endogeneous Livin protein (Figure 1b) and they reproducibly exhibited high DNA transfection efficiencies of more than 85% following calcium phosphate co-precipitation. After transfection, both pSUPER-Livin-1 and pSUPER-Livin-2 siRNAs efficiently inhibited endogeneous Livin protein expression (Figure 1c). This was not observed for the transfection control pSUPER-Luc, which specifically targets the non-cellular firefly *Photinus pyralis* luciferase gene for silencing (unpublished data). In contrast to Livin, the amounts of cellular Tubulin protein were not affected by either pSUPER-Livin-1 or pSUPER-Livin-2 (Figure 1c). In further support that Livin repression was caused by a specific siRNA effect, expression of both livin-1 and livin-2 siRNAs led to a clear reduction of livin transcripts in HeLa cells (Figure 1d). Taken together, these results demonstrate that vector-born siRNAs can specifically block livin gene expression in human cancer cells, with high efficiency.

### Example 2: Livin-2 siRNA increases caspase-3-like activities

Since pSUPER-Livin-2 regularly suppressed endogeneous Livin expression more strongly than pSUPER-Livin-1 (Figure 1), the former was chosen for subsequent analyses. It has been reported that Livin inhibits Caspase-3 activities following ectopic expression from heterologous promoters or in *in vitro* assays. In contrast to these studies, the siRNA approach followed in this study should allow to analyze the effects of endogeneous Livin on cellular Caspase-3 activities. As shown in Figure 2, pSUPER-Livin-2 increased Caspase-3-like activities in HeLa cells, indicating that the down-regulation of Livin expression is associated with a release of Caspase-3 from negative regulation by Livin. This conclusion is corroborated by the observation that the Caspase-3 inhibitor DEVD-fmk completely inhibited the increase of DEVD-cleavage following pSUPER-Livin-2 transfection in HeLa cells (Figure 2). In further support for the specificity of the Livin-targeting siRNAs, induction of caspase-3 activities by pSUPER-Livin-2 was observed in Livin-expressing HeLa cells, but not in H1299 cells (Figure 2) which do not express endogeneous Livin protein (Figure 1b).

### Example 3: Livin-2 siRNA increases caspase-3 in livin-positive cells treated with pro-apoptotic stimuli

It was investigated whether livin-2 siRNA can sensitize Livin-positive cells to pro-apoptotic stimuli. As shown in Figure 3, pSUPER-Livin-2 clearly increased the concentrations of the active form of Caspase-3 following treatment of HeLa cells with doxorubicine, u.v.-irradiation, and TNFα, when compared with control transfected cells (pSUPER-Luc). These events were associated with promoted cleavage of the caspase substrate Poly(ADP-ribose) polymerase (PARP) for all three pro-apoptotic agents, as indicated by the increase in the faster migrating PARP cleavage product, in the presence of pSUPER-Luc-2. Under the same experimental conditions, we did not observe a differential effect of pSUPER-Livin-2 on Caspase-7, -8, and -9 expression patterns, when compared with control transfected cells.

### Example 4: Livin-2 siRNA enhances apoptosis in livin-positive cells treated with pro-apoptotic stimuli

In order to analyze the cellular consequences of siRNA-mediated silencing of the livin gene, TdT-mediated dUTP biotin nick end labeling (TUNEL) analyses were performed, which allows the detection of apoptotic cells (Fig. 4a). In the absence of a pro-apoptotic stimulus, the number of TUNEL-positive cells increased approximately 2-fold following expression of pSUPER-Livin-2 (Figure 4a and b), indicating that siRNA directed against Livin modestly increases the spontaneous apoptosis rate of HeLa cells, in transient transfections. However, apoptosis stimulated by doxorubicin, u.v.-irradiation, and TNFα was strongly increased by pSUPER-Livin-2 in HeLa cells (Figure 4a and b). These results show that siRNA directed against Livin can strongly sensitize Livin-positive cancer cells towards pro-apoptotic stimuli.

### Example 5: Stable expression of Livin-2 siRNA abolishes colony-formation capacity of livin-expressing cells and induces their apoptosis

Colony formation assays were performed to analyze the effects of stable transfection of pSUPER-Livin-2 on the growth of tumor cells. To this end, HeLa cervical carcinoma, MeWo melanoma cells, and H1299 lung cancer cells were transfected with either pSUPER-Livin-2 or control vector pSUPER-Luc. In addition, pSV2Neo was cotransfected, to allow for the selection of transfected cells by neomycin resistance. As shown in Figure 5a, pSUPER-Livin-2 had a strong inhibitory effect on Livin-expressing HeLa cells, efficiently abolishing their colony formation capacity, when compared to control transfectants. This effect was specific for Livin-expressing cells, since it was also observed in MeWo cells, but not in Livin-H1299 cells (Figure 5a). In addition, HeLa cells stably transfected with pSUPER-Livin-2 exhibited a massive induction of apoptosis when compared to control-transfected cells, as visualized by a strong reduction in cell numbers, increased chromatin condensation, and a positive TUNEL assay (Figure 5b). These results show that the selection for stable pSUPER-Livin-2 transfectants results in the specific cell death of Livin-expressing cancer cells, through induction of apoptosis.

### MATERIALS AND METHODS

### Oligonucleotides and plasmids

Synthetic double-stranded oligonucleotides with the following sequences were introduced into vector pSUPER (Brummelkamp et al., 2002b): 5'-GTGGTTCCCCAGCTGTCAGttcaagagaCTGACAGCTGGGGAACCAC-3' (SEQ ID NO: 3, for livin-1),
5'-GGAAGAGACTTTGTCCACAttcaagagaTGTGGACAAAGTCTCTTCC-3' (SEQ ID NO:4, for livin-2). They consist of 19 nt sequences derived from the livin gene (nucleotide positions 611-629 for livin-1 and 648-666 for livin-2, numbering according to Kasof and Gomes, accession number AF311388) which are separated by a 9 nucleotide linker (lower case letters) from the reverse complement of the same 19 nt sequence. For negative control pSUPER-Luc, double-stranded oligonucleotide 5'-CATCACGTACGCGGAATACttcaagagaGTATTCCGCGTACGTGATG-3' (SEQ ID NO:5), which contains a 19 nt sequence complementary to the *P. pyralis* (variant GL3) luciferase gene, was introduced into pSUPER.

### Transfections, treatment with pro-apoptotic agents, and colony formation assays

HeLa, MeWo and H1299 cells were maintained in Dulbecco's minimal essential medium (pH 7.2), supplemented with 10% fetal calf serum. Exponentially growing HeLa cells were transfected by calcium-phosphate coprecipitation, yielding comparably high transfection efficiencies of over 85%, as assessed by transfecting a green fluorescent protein (GFP) producing vector (not shown). In transient transfections, cells were harvested 48 hours (h) after transfection for protein, caspase activity, and TUNEL analyses (see below). For u.v.-treatment, cells were exposed to a single dose of 50J per m² u.v. irradiation (Stratalinker 2400, Stratagene, Heidelberg, Germany) 36 h following transfection and harvested 12 h following irradiation. TNFα (Strathmann Boitec, Hamburg, Germany) was applied at a concentration of 1000 U/ml, in the presence of cycloheximide (Sigma, Taufkirchen, Germany); Doxorubicin (Sigma) was employed at a concentration of 0.5 µg/ml tissue culture medium.
For colony formation assays, cells were transfected with either pSUPER-Livin-2 or pSUPER-Luc, together with pSV2Neo, and selected for stably transfected cells by neomycin resistance, in the presence of 1 mg/ml Geneticin (Invitrogen). Colonies were fixed with formaldehyde and stained with crystal violet.

### RNA and protein analyses

For Northern blot analyses, poly-A⁺-RNA was isolated employing the Dynabeads mRNA DirectTM kit (Dynal, Oslo, Norway) according to the instructions provided by the manufacturer. Livin transcripts were detected using the complete radio-labeled livin-α cDNA as a probe. Glycerylaldehyde-3-phosphate dehydrogenase (GAPDH) served as a control probe to monitor equal loading between individual lanes.
Protein extracts were prepared essentially as described (Butz et al., 1999, Oncogene, Vol. 18, pages 2381-2386). For Western blot analyses, approximately 20 µg of protein was separated by 10% SDS-PAGE, transferred to a Immobilon-P membrane (Millipore, Bedford, USA) and analyzed by enhanced chemiluminiscence (Amersham, Braunschweig, Germany) using monoclonal anti-Livin antibody IMG347 (Biocarta Europe, Hamburg, Germany); monoclonal anti-Tubulin antibody Ab-1 (Oncogene, Boston, USA) , polyclonal rabbit anti-Caspase-3 antibody Pab CM1 (BD Biosciences, Heidelberg, Germany), polyclonal anti-Caspase-7 antibody (BD Biosciences), monoclonal anti-Caspase-8 antibody (BD Biosciences), polyclonal rabbit anti-Caspase-9 antibody (BD Biosciences), and mouse monoclonal anti-Poly(ADP-ribose) polymerase antibody C2-10 (BD Biosciences).

### Caspase and TUNEL assays

To detect caspase-3 protease activities, the ApoAlert Caspase-3 Colorimetric Assay Kit (Clontech, Palo Alto, USA) was utilized. Cytosolic lysates were prepared 48 h following transfection and incubated with 50 µM p-nitroanilide (pNA) conjugated to the caspase cleavage site Asp-Glu-Val-Asp (DEVD) for 1 h at 37° C. Hydrolyzed pNA was detected using a Multiscan MS colorimeter (ThermoLabsystems, Vantaa, Finland) at 405 nm. For control experiments, 10 µM of the Caspase-3 inhibitor DEVD-fmk (Clontech) was included into the reaction, before addition of the substrate.
For apoptosis detection, cells were grown on coverslips and TdT-mediated dUTP biotin nick end labeling (TUNEL) analyses were performed using the *in situ* cell death detection kit (Roche Molecular Biochemicals), as previously described (Butz et al., 2000, Proc. Natl. Acad. Sci., Vol. 97, pages 6693-6697). Total DNA was stained with 4',6-diamidino-2-phenylindole (DAPI, Roche Molecular Biochemicals). Apoptotic strand breaks and total DNA were visualized by transmission epifluorescence microscopy.

## Claims

1. The use of a nucleic acid containing the sequence of SEQ ID NO:1, a fragment or derivative thereof, or SEQ ID NO:2, a fragment or derivative thereof, to prepare siRNA which sensitizes therapy-resistant tumor cells for apoptosis.

2. The use according to claim 1, wherein said nucleic acid has a length of 15 to 25 nucleotides, preferably 18 to 22 nucleotides and most preferably 19 nucleotides.

3. The use according to claims 1 or 2, wherein the siRNA is delivered into a therapy-resistant tumor cell.

4. The use according to claim 3, wherein the delivery is done by using liposomes or hydrodynamic injection.

5. The use of a nucleic acid containing the sequence of SEQ ID NO:3, a fragment or derivative thereof, or SEQ ID NO:4, a fragment or derivative thereof, to prepare siRNA which sensitizes therapy-resistant tumor cells for apoptosis.

6. The use according to claim 5, wherein the nucleic acid is inserted into an expression vector.

7. The use according to claim 6, wherein the expression vector allows for the production of dsRNA.

8. The use according to claim 6 or 7, wherein the expression vector is pSUPER.

9. An expression vector containing the sequence of SEQ ID NOs:3, a fragment or derivative thereof, or SEQ ID NO:4, a fragment or derivative thereof.

10. A method for down-regulating livin in a therapy-resistant tumor cell comprising contacting the cell with a siRNA containing the sequence of SEQ ID NOs: 1, 2, 3, and/or 4.

11. The use of a siRNA containing the sequence of SEQ ID NO:1, 2, 3, or 4, as a pharmaceutical for the treatment of therapy-resistant tumors.

12. The use according to claim 11, wherein the siRNA is used in combination with radiation therapy.

13. The use according to claim 11, wherein the siRNA is used in combination with an active compound which is selected from the group consisting of cytostatic compounds, death receptor ligands, antibodies to death receptors and negative regulators of anti-apoptotic proteins.

14. The use of a siRNA containing the sequence of SEQ ID NO:1, 2, 3, or 4, for the manufacture of a medicament for the treatment of therapy-resistant tumors.

15. The use according to any of claims 11 to 14, wherein the therapy-resistant tumor is selected from the group consisting of neuroblastoma, intestine carcinoma preferably rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, follicular thyroid carcinoma, anaplastic thyroid carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors preferably glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

16. The use according to any of claims 11 to 15, wherein the therapy-resistant tumor is cervical carcinoma or melanoma.

17. A medicament for the treatment of therapy-resistant tumors comprising a siRNA containing the sequence of SEQ ID NOs:1, 2, 3, or 4, a pharmaceutically acceptable carrier and, optionally, an active compound.
